# EUROPEAN PATENT APPLICATION

(11) **EP 3 915 594 A1**
(43) Date of publication of application: **01.12.2021**
(21) Application number: 21170933.2
(22) Date of filing: 28.04.2021
(51) Int. Cl.: A61L 2/10

(54) **STERILIZATION TOOL FOR DENTAL AND MEDICAL TOOLS AND RELATED SYSTEMS AND METHODS**

(30) Priority: 07.05.2020 US 202063021529 P
(71) Applicant: Lazurtec, Inc., Mesa, AZ 85205 (US)
(72) Inventor: DEBRAY, Jean-Philippe, Mesa, Arizona, 85207 (US); ARENA, Chantal, San Jose, California, 95131 (US); FOSTER, Robert, Mesa, Arizona, 85205 (US)
(74) Representative: IP Trust

(57) **Abstract**

A sterilization apparatus may include a cavity defined by walls of the apparatus. The sterilization apparatus may further include at least one internal compartment within the cavity. The internal compartment may include at least one reflective surface. The sterilization apparatus may also include one or more ultraviolet lights on a surface of at least one of the walls of the apparatus and directed toward the at least one internal compartment. The one or more ultraviolet lights may be configured to produce ultraviolet light in one or more of the UVB and UVC spectrums.

## Description

### CROSS-REFERENCE TO RELATED APPLICATION

This application claims the benefit under 35 U.S.C. §119(e) of U.S. Provisional Patent Application Serial No. 63/021,529, filed May 7, 2020, for "Sterilization Tool For Dental And Medical Tools And Related Systems And Methods," the disclosure of which is hereby incorporated herein in its entirety by this reference.

### TECHNICAL FIELD

Embodiments of the present disclosure generally relate to sterilization tools. In particular, embodiments of the present disclosure relate to sterilization tools for dental and medical tools and related systems and methods.

### BACKGROUND

Many tools used in medical fields, such as doctor's offices, hospitals, and dentist offices, must be sterilized between each use. Sterilizing the tools may remove bacteria, viruses, and other micro-organisms that may cause disease and may be otherwise passed between patients by the respective tools. Sterilizing processes are generally required in the different medical fields between each use of the respective tools. In some instances, medical tools are single use disposable tools to avoid the need to sterilize the tools. However, some medical tools are expensive complex tools that must be sterilized after every use. Complex geometry of the tools may affect the types of sterilization processes that may be used. Complex geometry of the tools may also require greater amounts of time to effectively sanitize the entire tool

### DISCLOSURE

Embodiments of the present disclosure may include a sterilization apparatus. The sterilization apparatus may include a cavity defined by walls of the apparatus. The sterilization apparatus may further include at least one internal compartment within the cavity. The internal compartment may include at least one reflective surface. The sterilization apparatus may also include one or more ultraviolet lights on a surface of at least one of the walls of the apparatus and directed toward the at least one internal compartment. The one or more ultraviolet lights may be configured to produce ultraviolet light in one or more of the UVB and UVC spectrums.

Another embodiment of the present disclosure may include a sterilization appliance. The sterilization appliance may include a bottom surface; a lid; one or more walls; and a cavity defined by the bottom surface, the lid, and the one or more walls. The sterilization appliance may further include at least one large compartment positioned within the cavity and one or more small compartments positioned within the cavity. The sterilization appliance may also include a plurality of UV light sources positioned on at least one of the bottom surface, the lid, or the one or more walls, the plurality of UV lights positioned to correspond with at least one of the at least one large compartment and the one or more small compartments.

Another embodiment of the present disclosure may include a method of sterilizing a tool. The method may include suspending the tool in an internal compartment. The compartment may include a UV reflective surface. The method may further include closing a lid over the internal compartment. The method may also include supplying UV radiation to the internal compartment at a beam angle configured to reflect multiple times from the UV reflective surface of the compartment

### BRIEF DESCRIPTION OF THE DRAWINGS

While the specification concludes with claims particularly pointing out and distinctly claiming embodiments of the present disclosure, the advantages of embodiments of the disclosure may be more readily ascertained from the following description of embodiments of the disclosure when read in conjunction with the accompanying drawings in which:
FIG. 1 illustrates a graphical representation of the germicidal effects of different wavelengths of ultraviolet radiation;
FIGS. 2 - 4 illustrate perspective views of a sterilization appliance according to an embodiment of the present disclosure;
FIGS. 5A and 5B illustrate perspective views of embodiments of the internal cavity of the embodiment of the sterilization appliance illustrated in FIGS. 2-4;

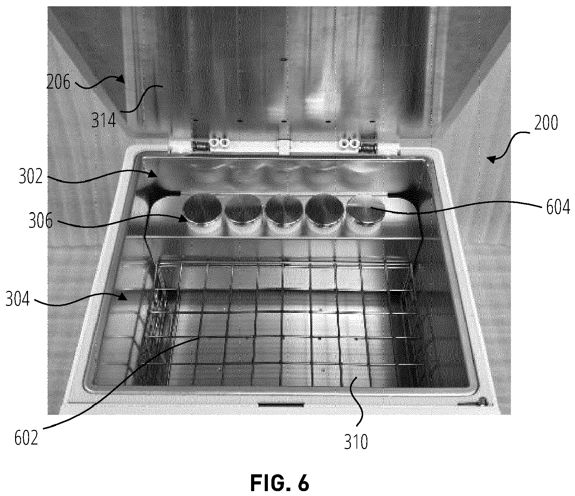

FIG. 6 illustrates a view of the internal cavity of an embodiment of the sterilization appliance illustrated in FIGS. 2-4;
FIGS. 7A -7D illustrate embodiments of caps and tools associated with the sterilization appliance illustrated in FIGS. 2-4;
FIG. 8 illustrates a schematic views of an embodiment of a compartment of the sterilization appliance illustrated in FIGS. 2-4;
FIG. 9 illustrates a schematic view of a compartment of the sterilization appliance illustrated in FIGS. 2-4;
FIGS. 10A and 10B illustrate perspective views of LED surface mount packages according to embodiments of the present disclosure; and
FIGS. 11A-11C illustrate side views of LED surface mount packages according to embodiments of the present disclosure.

### MODE(S) FOR CARRYING OUT THE INVENTION

The illustrations presented herein are not meant to be actual views of any particular sterilization apparatus, system, or component thereof, but are merely idealized representations employed to describe illustrative embodiments. The drawings are not necessarily to scale.

As used herein, the singular forms following "a," "an," and "the" are intended to include the plural forms as well, unless the context clearly indicates otherwise.

As used herein, the term "may" with respect to a material, structure, feature, or method act indicates that such is contemplated for use in implementation of an embodiment of the disclosure, and such term is used in preference to the more restrictive term "is" so as to avoid any implication that other compatible materials, structures, features, and methods usable in combination therewith should or must be excluded.

As used herein, any relational term, such as "first," "second," "top," "bottom," "upper," "lower," "above," "beneath," "side," "upward," "downward," etc., is used for clarity and convenience in understanding the disclosure and accompanying drawings, and does not connote or depend on any specific preference or order, except where the context clearly indicates otherwise. For example, these terms may refer to an orientation of elements of a grill device tem when utilized in a conventional manner. Furthermore, these terms may refer to an orientation of elements of a grill device as illustrated in the drawings.

As used herein, the term "substantially" in reference to a given parameter, property, or condition means and includes to a degree that one skilled in the art would understand that the given parameter, property, or condition is met with a small degree of variance, such as within acceptable manufacturing tolerances. By way of example, depending on the particular parameter, property, or condition that is substantially met, the parameter, property, or condition may be at least 90.0% met, at least 95.0% met, at least 99.0% met, or even at least 99.9% met.

As used herein, the term "about" used in reference to a given parameter is inclusive of the stated value and has the meaning dictated by the context (e.g., it includes the degree of error associated with measurement of the given parameter, as well as variations resulting from manufacturing tolerances, etc.).

As used herein, the term "and/or" means and includes any and all combinations of one or more of the associated listed items.

As used herein, the terms "vertical" and "lateral" refer to the orientations as depicted in the figures.

Sanitizing medical tools can be a time consuming process involving chemicals and/or high temperatures to remove the bacteria and viruses from the respective tools. The chemicals and high temperatures may cause the tools to break down over time reducing the operating life of the tools.

Ultraviolet (UV) light can be used to remove bacteria and viruses from air, water, and physical surfaces. UV light is a spectrum of light having wavelengths of between about 100 nanometers (nm) and about 400 nm. The UV spectrum may be further separated into three spectrums, UVA having wavelengths of between about 315 nm and about 400 nm, UVB having wavelengths of between about 280 nm and about 315 nm and UVC having wavelengths of between about 100 nm and about 280 nm. Light in the UVC & UVB spectrums may be absorbed by the DNA and RNA of bacterial microorganisms, such as bacteria and viruses, including SARS virus and more generally Coronaviruses. The UVC and/or UVB light may alter the DNA and RNA of the associated microorganism effectively destroying the organism. Thus UV radiation may sterilize and/or disinfect the environment (water, air, surfaces), while causing minimal damage to the associated surface, such as metal and/or polymer surfaces used in medical tools.

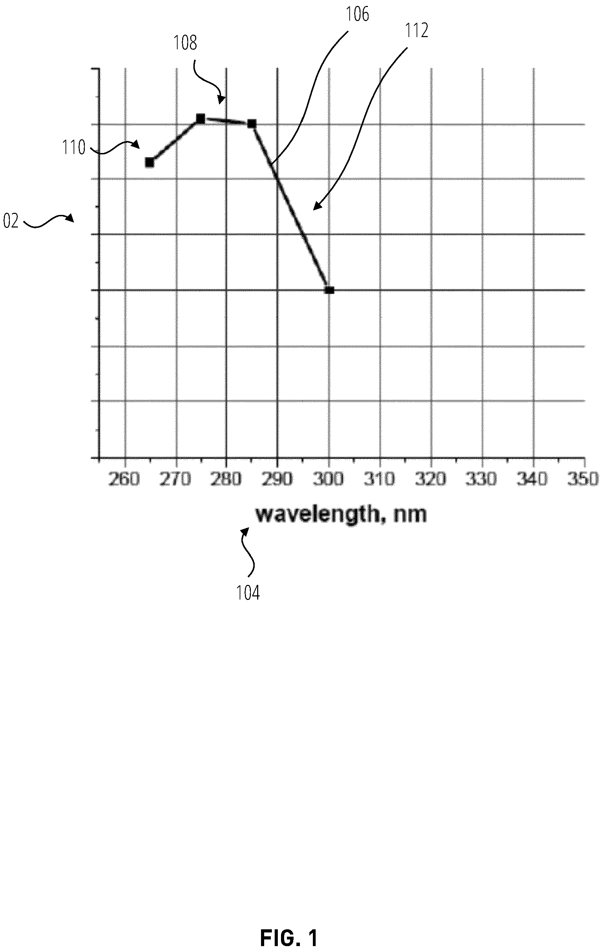

FIG. 1 illustrates a graphical representation of the germicidal effect 102 of a dose 106 of UV radiation at different wavelengths 104. For sanitation and sterilization, the dose 106 of UV radiation may be provided at wavelengths within the UVC and/or UVB spectrums, such as between about 100 nm and about 315 nm, or between about 200 nm and about 315 nm.

As illustrated in the graphical representation, a particular dose 106 of UV radiation may have wavelengths 104 that provide high efficiency 108 germicidal effects 102, moderate efficiency 110 germicidal effects 102, and low efficiency 112 germicidal effects 102. The dose 106 of UV radiation may be associated with an intensity of the UV radiation and the amount of time that the UV radiation is supplied. Thus, increasing the intensity of the UV radiation or the amount of time that the UV radiation is supplied may increase the dose 106. Increasing the dose of UV radiation may increase the germicidal effect 102. As the efficiency of the germicidal effect 102 of the dose 106 increases, the dose 106 required to sterilize an item (e.g., substantially remove all bacteria and viruses from the item) may be reduced.

As illustrated in the graphical representation, UV radiation within the UVC spectrum may provide a high efficiency in sterilization.

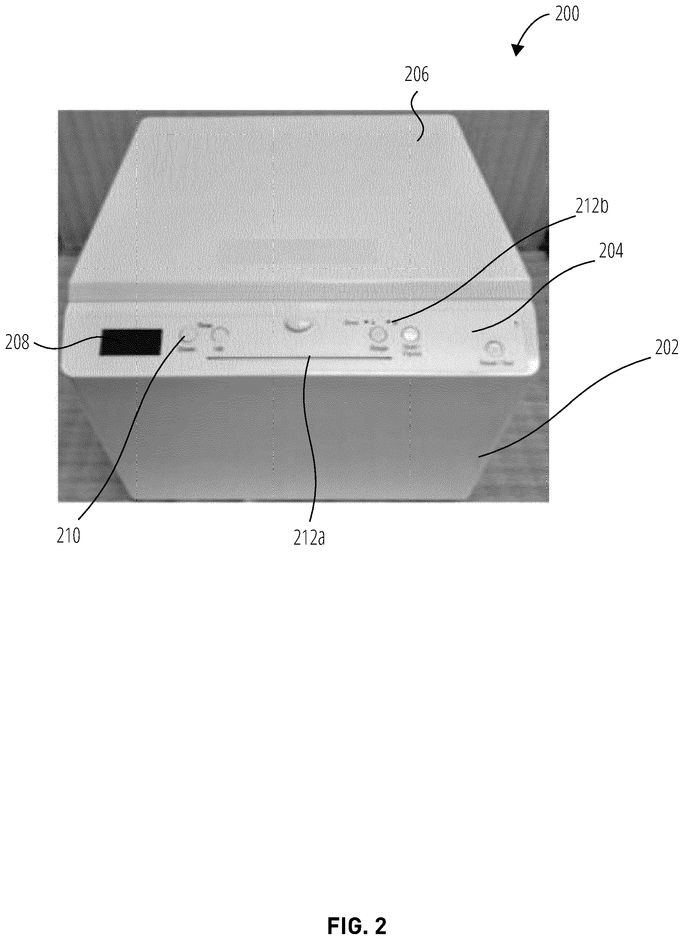

FIG. 2 illustrates an embodiment of a sterilization apparatus 200 for sterilization or disinfection of small devices, such as medical tools or dental tools. The sterilization apparatus 200 may include a box 202 containing various compartments designed to optimize and increase the UV light distribution and reflection within the various compartments, as described in further detail below. The sterilization apparatus 200 may further include a control section 204 configured to control the distribution of UV light within the box 202.

The box 202 may be formed from a polymer material, such as acrylonitrile butadiene styrene (ABS). The box 202 may have dimensions that are large enough to receive common items to be sterilized, such as common medical tools or dental tools, while remaining small enough to be placed on a counter or shelf in an office. For example, the box 202 may have a length and/or width of between about 100 millimeters (mm) and about 300 mm, such as between about 180 mm and about 220 mm and a height of between about 100 mm and about 200 mm, such as about 130 mm.

The box 202 may include a lid 206. The lid 206 may enable a user to insert the devices to be sterilized into the box 202 and/or remove the sterilized devices from the box 202 after the sterilization process. In some embodiments, the lid 206 may be coupled to the box 202 through a hinged connection. In other embodiments, the lid 206 may be configured to be lifted completed from a top surface of the box 202. In some embodiments, the lid 206 may include a locking latch. The locking latch may be configured to electronically lock the lid 206 closed, such as during a sanitization process. Locking the lid 206 in a closed position may enable the sanitization process to proceed without being interrupted by opening the lid, such that the dosage of UV light within the box 202 may be accurately controlled during the sanitization process.

The control section 204 may include a display 208, user input elements 210, and lights 212a, 212b. The display 208 may provide information to the user. The display 208 may be a segment display (e.g., seven-segment display), a light-emitting diode (LED) display, liquid crystal display (LCD), electroluminescent display (ELD), plasma display (PDP), quantum dot display (QLED), etc. In some embodiments, the display 208 may be a graphic user interface configured to display information and receive user input. The display 208 may provide the user with information regarding the operation of the sterilization apparatus 200, such as status, settings, elapsed time, time remaining, errors, failures, etc.

The control section 204 may include multiple different user input elements 210, such as buttons, dials, switches, sliders, etc. The user input elements 210 may enable the user to change settings, start functions, stop processes, change programming, select functions, etc. For example, the user input elements 210 may enable a user to change timers, set an intensity of the UV lights, change a dosage of the UV light, turn the UV lights on and off, start a sanitization process, select a compartment to sanitize, adjust a time for the sanitization process, etc.

The control section 204 may also include different lights 212a, 212b. The lights 212a, 212b may be indicator lights 212b, such as on/off lights. For example, the lights 212b may turn on to indicate that the UV lights within the box 202 are on. In some cases, the lights 212b may be configured to indicate which selection has been made by the user, such as which compartment will be sanitized, which preprogrammed settings will be used, an intensity level (e.g., high, medium, low), a door latch indicator, etc. Some of the lights 212a, 212b, may be a light bar 212a. The light bar 212a may display a long thin light. In some embodiments, the light bar 212a may modulate, such as a progress bar. For example, the light bar 212a may gradually increase in length until the entire light bar 212a is lit to indicate progress of a sanitization process. In some embodiments, one or more of the lights 212a, 212b may provide status information, such as on/off status, errors, etc. For example, the lights 212a, 212b may light up in different colors, or patterns to provide status information to a user.

The sterilization apparatus 200 may include safety features in control section 204 of the sterilization apparatus 200. The safety features may include an automatic warning alarm and switch-off of the LEDs if the cover lid is not shut, an automatic warning alarm if the LEDs are shunt, an automatic alert if the light intensity of the LEDs is below 70% of the initial or nominal output power. In some embodiments, the sterilization apparatus 200 may be associated with an automated process configured to load and/or unload the tools to/from the sterilization apparatus 200 with minimal to no user interaction.

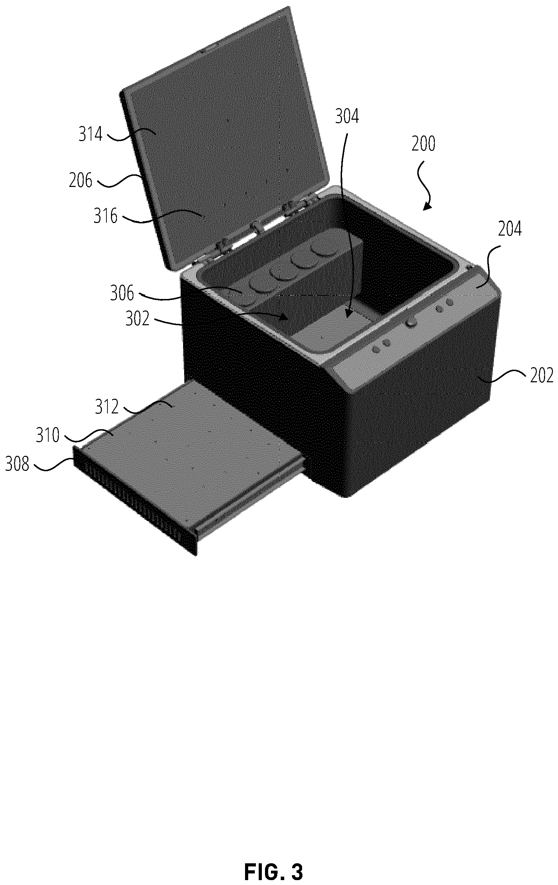

FIG. 3 illustrates the sterilization apparatus 200 in an open configuration. A cavity 302 may be defined within the box 202. The cavity 302 may include multiple compartments, such as a large compartment 304 and/or one or more small compartments 306. The compartments 304, 306 may have interior surfaces formed from UV reflecting materials, such as stainless steel (e.g., SUS 204, SAE 204 stainless steel, SS304, etc.), polytetrafluoroethylene (e.g., PTFE, Teflon, etc.), quartz, etc. The compartments 304, 306 may be configured to receive items to be sanitized, such as medical tools, dental tools, etc. UV lights may be positioned within the compartments 304, 306 to impinge UV radiation on the surfaces of any items positioned in the compartments 304, 306.

The sterilization apparatus 200 may include a bottom light panel 310 configured to provide the UV radiation to the compartments 304, 306. The bottom light panel 310 may be located on a bottom surface of the cavity 302. The sterilization apparatus 200 may include a drawer 308 configured to define the bottom surface of the cavity 302, such that the bottom light panel 310 may be removed for cleaning, servicing, repairs, etc. The bottom light panel 310 may include multiple UV lights 312. For example, the bottom light panel 310 may include at least one UV light 312 for each compartment 304, 306. In some embodiments, the bottom light panel 310 may include more than one UV light 312 for each compartment 304, 306. For example, the bottom light panel 310 may include at least one UV light 312 for each of the small compartments 306 and may include multiple UV lights 312 for the large compartment 304, such as between about 5 UV lights 312 and about 20 UV lights 312, between about 10 UV lights 312 and about 15 UV lights 312, or about 10 UV lights 312.

The sterilization apparatus 200 may further include a top light panel 314 configured to provide the UV radiation to the compartments 304, 306. The top light panel 314 may be located on an interior surface of the lid 206. The interior surface of the lid 206 may form a top surface of the cavity 302. Opening the lid 206 may enable a user to access the top light panel 314 and/or remove the top light panel 314 for cleaning, servicing, repairs, etc. The top light panel 314 may include multiple UV lights 316. For example, the UV lights 316 may be arrange in substantially the same arrangement as the UV lights 312 on the bottom light panel 310. In some embodiments, the UV lights 316 on the top light panel 314 may be arranged in different positions relative to the bottom light panel 310. For example, arranging the UV lights 316 on the top light panel 314 differently from the UV lights 312 on the bottom light panel 310 may provide greater coverage throughout the cavity 302 and the compartments 304, 306. Similar to the bottom light panel 310, the top light panel 314 may include at least one UV light 316 for each compartment 304, 306. In some embodiments, the top light panel 314 may include more than one UV light 316 for each compartment 304, 306. For example, the top light panel 314 may include at least one UV light 316 for each of the small compartments 306 and may include multiple UV lights 316 for the large compartment 304, such as between about 5 UV lights 316 and about 20 UV lights 316, between about 10 UV lights 316 and about 15 UV lights 316, or about 10 UV lights 316.

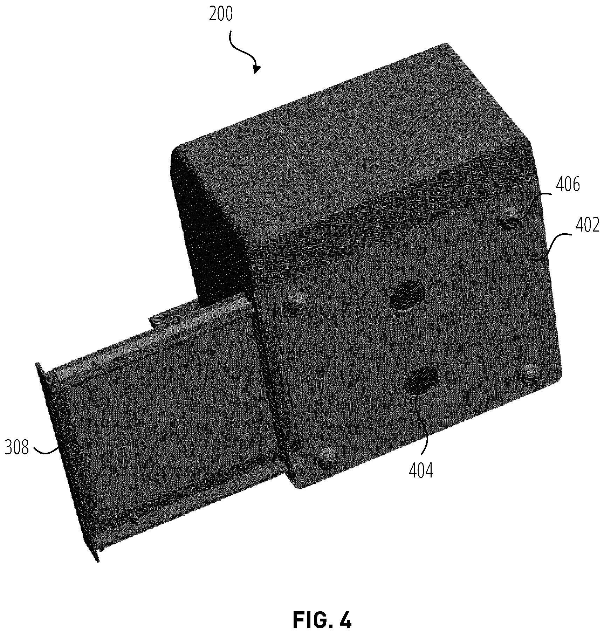

FIG. 4 illustrates a view of a bottom 402 of the sterilization apparatus 200. The sterilization apparatus 200 may include one or more vents 404. The vents 404 may be configured to cool internal components of the sterilization apparatus 200. For example, the vents 404 may enable airflow over electronic components, cooling fins, heat sinks, etc., of internal electronics of the sterilization apparatus 200. In some embodiments, the sterilization apparatus 200 may include one or more ventilation fans associated with the vents 404 configured to create air movement through the vents 404. In some embodiments, the vents 404 may be configured as a supply vent and a relief vent, such that air flow may enter the sterilization apparatus 200 through the supply vent and exit through the relief vent passing over the electronic components and/or cooling elements (e.g., heat sinks, cooling fins, etc.) while moving between the supply vent and the relief vent.

The drawer 308 may be positioned between the vents 404 and the cavity 302. In some embodiments, the drawer 308 may be configured to create a seal at a bottom of the cavity 302, such that air entering through the vents 404 may be substantially prevented from entering the cavity 302. For example, the electronics and/or other elements being cooled by the airflow through the vents 404 may be positioned in a space between the drawer 308 and the bottom 402 of the sterilization apparatus 200, such that airflow through the vents 404 may pass over the elements being cooled without entering the cavity 302.

The bottom 402 of the sterilization apparatus 200 may include feet 406. The feet 406 may be configured to support the sterilization apparatus 200 and create a space between the bottom 402 of the sterilization apparatus 200 and a surface on which the sterilization apparatus 200 is placed. The space may enable airflow into the vents 404 and out of the vents 404.

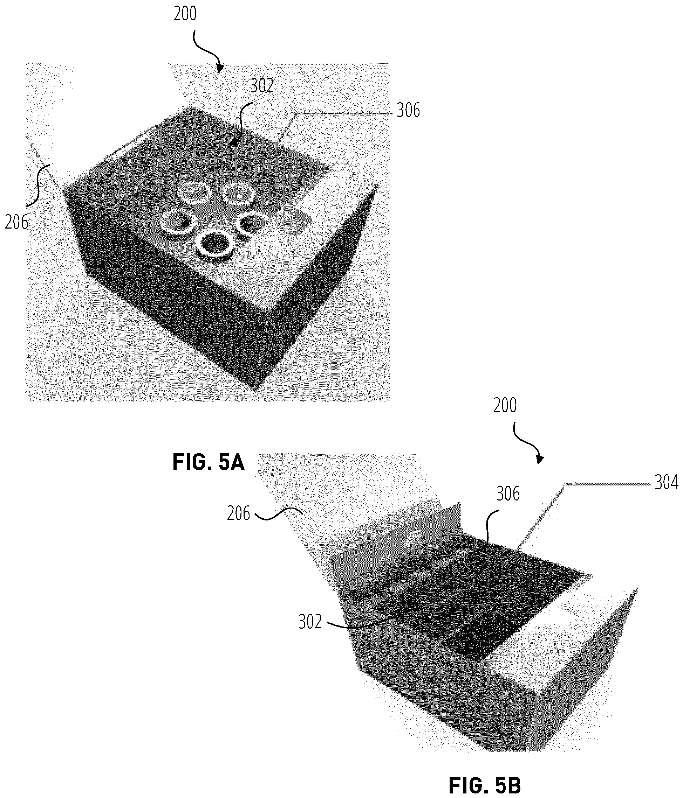

FIG. 5A and FIG. 5B illustrate embodiments of the sterilization apparatus 200 having different configurations of compartments 304, 306 within the cavity 302. In some embodiments, the sterilization apparatus 200 may include small compartments 306 within the cavity 302 and no large compartment 304, as illustrated in FIG. 5A. The small compartments 306 may be arranged in a circular (e.g., annular) configuration. In some embodiments, the small compartments 306 may be arranged in multiple annular rings. In some embodiments, the small compartments 306 may be arranged in linear rows. For example, the cavity 302 may include multiple linear rows forming an array of small compartments 306 throughout the cavity 302.

In some embodiments, the small compartments 306 may have different sizes and/or shapes to accommodate different elements. For example, some of the small compartments 306 may have larger cross-sectional areas than other small compartments 306 to accommodate larger elements. In some cases, some of the small compartments 306 may have circular cross-sections, such as cylindrical tubes, or conical tubes, and others may have cross-sections having another shape, such as square, triangular, polyhedral, etc., (*e.g.,* pyramidal tubes, rectangular prism tubes, polyhedron tubes, *etc.*)*,* to accommodate elements having different shapes. The small compartments 306 may be arranged in a vertical position (e.g., such that an axis of the small compartments 306 is vertical). The small compartments 306 may also be arranged to provide an axial line of sight for at least one of the UV lights 312, 316 into each of the small compartments 306.

FIG. 5B illustrates a sterilization apparatus 200 having a large compartment 304 and multiple small compartments 306 arranged within the cavity 302. In some embodiments, the sterilization apparatus 200 may include a large compartment 304 and one small compartment 306, such as the area where the small compartments 306 are positioned in FIG. 5B without the individual cylindrical small compartments 306.

The large compartment 304 in the cavity 302 of the sterilization apparatus 200 may include a support 602. The support 602 may be configured to suspend the common items to be sterilized, such as medical tools or dental tools within the large compartment 304, such that the items being sterilized do not rest on the bottom surface of the cavity 302. Suspending the items being sterilized above the bottom surface of the cavity 302 may enable UV radiation from the UV lights 312 in the bottom light panel 310 and the UV lights 316 in the top light panel 314 to have greater coverage and impinge on the surfaces of the item being sterilized. The support 602 may be positioned a distance of between about 1 mm and about 100 mm from the bottom light panel 310. The distance may depend on the UV light emission angle of the UV lights 312, 316 in the bottom light panel 310 and the top light panel 314.

The support 602 may be formed in a way that does not substantially interrupt the UV radiation from the UV lights 312, 316. For example, the support 602 may be formed from a screen, such as an array of intersecting thin metal rods forming a screen leaving large uninterrupted areas between the intersecting metal rods through which the UV radiation may pass. In other embodiments, the support 602 may be formed from a material that is substantially UV transparent, such as UV transparent glass (e.g., quartz, fused silica, etc.), UV transparent polymers (e.g., acrylic), and UV transparent ceramics.

The small compartments 306 may include caps 604 positioned on a top portion of the small compartments 306. The caps 604 may be configured to suspend an item to be sterilized in the associated small compartment 306.

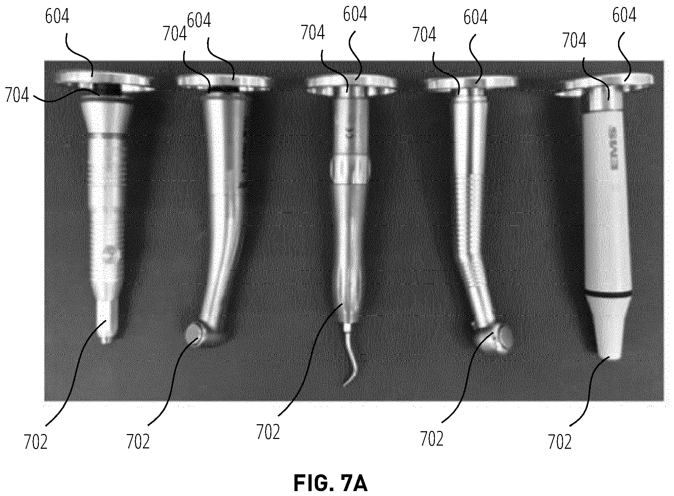

FIGS. 7A - 7D illustrate several caps 604 suspending tools 702 therefrom. The caps 604 may include one or more types of couplers configured to interface with the tools 702. For example, the couplers may be common tool couplers, such as threaded connections, quick release connections (e.g., ball-latching, bayonet threaded, etc.), twist connections, etc.

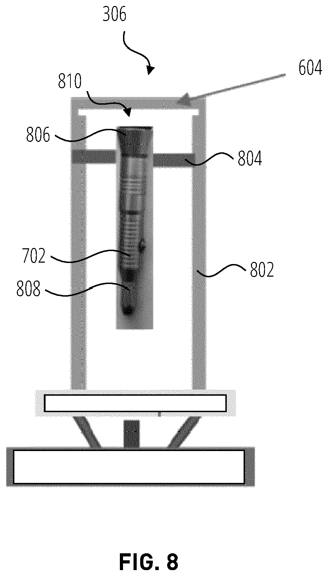

In some embodiments, the caps 604 may be formed from a UV transparent material, such as UV transparent glass (e.g., quartz, fused silica, etc.), UV transparent polymers (e.g., acrylic), or UV transparent ceramics. For example, the caps 604 may be formed from a UV transparent material, such that UV radiation from the associated UV lights 316 (FIG. 3) in the top light panel 314 (FIG. 3) may enter the associated small compartment 306 through the cap 604. In other embodiments, the caps 604 may be formed from a UV reflective material, such as UV reflective polymers (e.g., PTFE, TEFLON®, etc.), or metal materials (e.g., stainless steel, aluminum, nickel, titanium, chromium, gold, etc.). For example, the caps 604 may be formed from a UV reflective material, such that UV radiation from the UV lights 312 in the bottom light panel 310 may substantially remain within the small compartment 306 as described in further detail below with respect to FIG. 8.

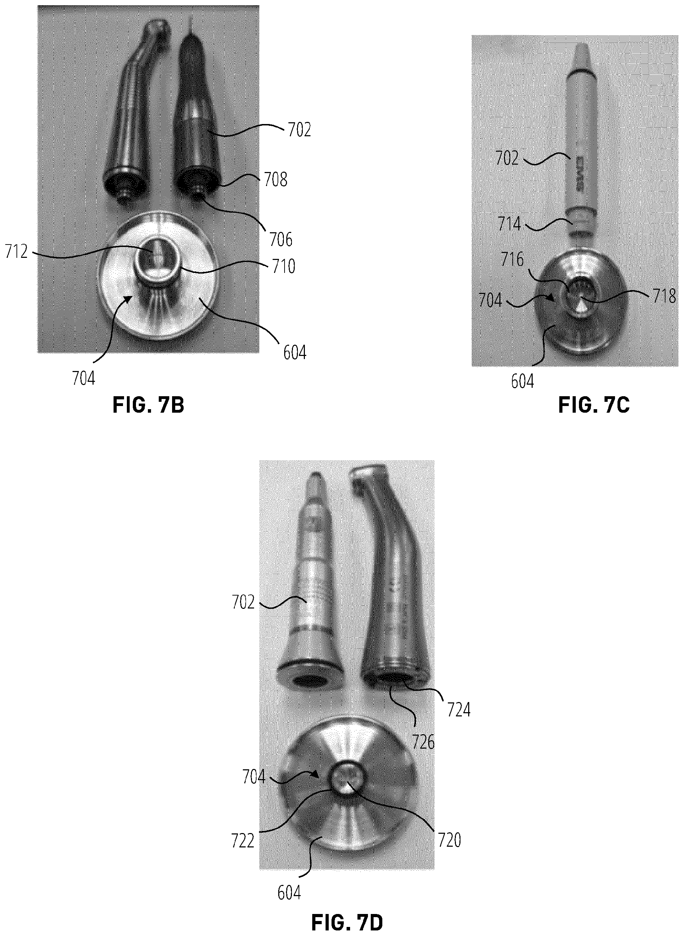

FIG. 7B illustrates an embodiment of a coupler 704 on a cap 604. The associated tool 702 may include a protrusion 706 substantially centrally (e.g., axially) positioned relative to the tool 702. For example, the protrusion 706 may be a drive element configured to be operatively coupled to a motorized handle, such as for a dental drill. The protrusion 706 may be surrounded by an internal interface 708, such as threads, a quick connect fittings (e.g., ball-latching, bayonet threaded, etc.). The internal interface 708 may be positioned within the tool 702, such as on an inner wall of the tool 702.

The coupler 704 on the cap 604 may include complementary features. For example, the coupler 704 may include a recess 712 configured to receive the protrusion 706 of the tool 702. The recess 712 may be surrounded by a complementary external interface 710. The complementary external interface 710 may be complementary to the internal interface 708 of the tool 702. For example, if the internal interface 708 of the tool 702 is a threaded interface, the complementary external interface 710 may include complementary threads. If the internal interface 708 of the tool 702 is a ball-latching quick connect fitting, the complementary external interface 710 may be a cylindrical geometry, such as an annular ridge, annular seal, snap ring, etc., configured to interface with the ball-latching quick connect fitting. The complementary external interface 710 may be configured to be disposed within the tool 702 and secure the tool 702 to the cap 604 through the internal interface 708.

FIG. 7C illustrates an embodiment of a coupler 704 on a cap 604. The associated tool 702 may include an external interface 714 over a portion of the outer surface of the tool 702. The external interface 714 may include threads, quick-connect geometry (e.g., annular ridges, recesses, seals, snap rings, etc.), or other elements configured to interface with another complementary part, such as a handle, water supply tube, air supply tube, light supply device, motor, etc., to couple the tool 702 to the complementary part.

The coupler 704 on the cap 604 may include complementary features for securing the tool 702 to the cap 604. For example, the coupler 704 may include a recess 718 configured to receive the portion of the tool 702 having the external interface 714. The recess 718 may include a complementary internal interface 716 configured to couple the cap 604 to the tool 702 through the external interface 714 on the outer surface of the tool 702.

FIG. 7D illustrates another embodiment of a coupler 704 on a cap 604. The tool 702 may include a cavity 724 defined in an end of the tool 702. The cavity 724 may include an internal interface 726, such as threads, quick-connect fittings, etc. The cavity 724 may be configured to receive a complementary part, such as a handle, water supply tube, air supply tube, light supply device, motor, etc., to couple the tool 702 to the complementary part.

The coupler 704 on the cap 604 may include complementary features for securing or holding the tool 702 to the cap 604. For example, the coupler 704 may include a protrusion 720 configured to be disposed within the cavity 724 of the tool 702. The protrusion 720 may include a complementary external interface 722 configured to couple to cap 604 to the tool 702 through the internal interface 726 in the cavity 724.

FIG. 8 illustrates an embodiment of a small compartment 306. The small compartment 306 may be configured to suspend the tool 702 within the small compartment 306 through a support 804 extending from a wall 802 of the small compartment 306. For example, the support 804 may include one or more support structures extending from the wall 802 of the small compartment 306 to contact an outer surface of the tool 702. For example, the tool 702 may have a proximal portion 806 having a larger major cross-sectional dimension (e.g., diameter, radius, apothem, width, etc.) than a major cross-sectional dimension of a distal portion 808 of the tool 702. Thus, an opening in the support 804 may be sized such that the distal portion 808 of the tool 702 may pass through the opening and the proximal portion 806 may be larger than the opening, such that the proximal portion 806 rests on the support 804 and the distal portion 808 of the tool 702 is suspended below the support 804.

The tool 702 may not be coupled to the cap 604, such that a void 810 may be defined between the tool 702 and the cap 604. The void 810 may enable UV radiation to impinge on portions of the proximal portion 806 of the tool 702 that may be covered if the tool 702 is coupled to the cap 604.

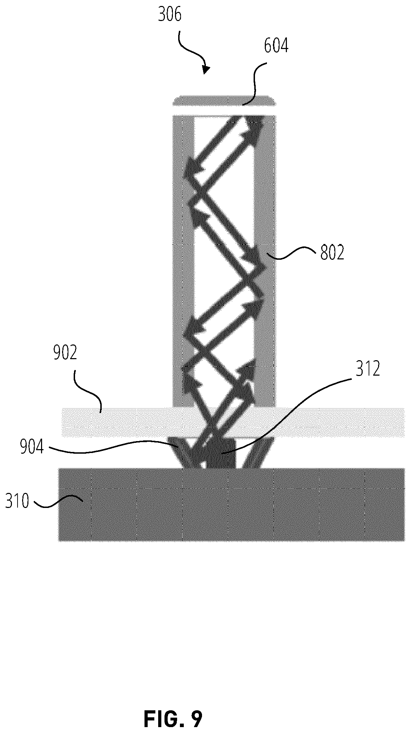

FIG. 9 illustrates an embodiment of a small compartment 306. The small compartment 306 may be positioned over the bottom light panel 310 as discussed above in relation to FIG. 3. The bottom light panel 310 may include at least one UV light 312 associated with the small compartment 306. In some embodiments, the UV light 312 may be one or more UV LED lights, such as a UVB LED light, a UVC LED light, an array of UVB LED lights, or an array of UVC LED lights.

A cover material 902 may be positioned between the UV light 312 and the small compartment 306. The cover material 902 may be formed from a UV transparent material, such as UV transparent glass (e.g., quartz, fused silica, etc.), UV transparent polymers (e.g., acrylic), or UV transparent ceramics. In some embodiments, the cover material 902 may be configured to act as an optical device to spatially distribute or concentrate the light source according to a desired pattern.

The light may enter the small compartment 306 may passing through the cover material 902. The walls of the small compartment 306 may act as a light pipe. For example, the walls 802 of the small compartment 306 may include a UV reflective material, such as UV reflective polymers (e.g., PTFE, TEFLON®, etc.), or metal materials (e.g., stainless steel, aluminum, nickel, titanium, chromium, gold, etc.). The UV reflective material, may cause the light to reflect between the walls 802 of the small compartment 306 as illustrated in FIG. 9. Reflecting the light between the walls 802 may create a large distribution of light such that a tool 702 (FIGS. 7A-7D) suspended in the small compartment 306 may impinge on the tool 702 over a broader area than a single beam of light. A large distribution of light may improve the coverage of the UV radiation within the small compartment 306. Thus, the large distribution of light may provide a more consistent dose of UV radiation across the entire surface of the tool 702. The large distribution of light may decrease an amount of time required to provide an effective dose of UV radiation to substantially the entire surface of the tool. For example, an effective dose at least about 43 millijoules/cm² may be applied in less than about 5 minutes, such as less than about 3 minutes, or less than about 1 minute.

As described above, the cap 604 may be formed from a UV reflective material similar to the walls 802 of the small compartment 306. The UV reflective material on the cap 604 may cause the UV radiation within the small compartment 306 to reflect back into the small compartment 306 once the UV radiation reaches a top portion of the small compartment 306. Thus, the UV radiation may continue to act on the surface of the tool 702 rather than being lost through the cap 604. Furthermore, the reflected UV radiation may travel in a different path through the small compartment 306 impinging on different portions of the tool 702.

UV reflective mirrors 904 may be included around the plane of the UV light 312. The mirrors 904 may increase light reflection efficiency. For example, as UV radiation exits the small compartment 306 through the cover material 902 at the bottom of the small compartment 306, the mirrors 904 may reflect the UV radiation back into the small compartment 306 enabling the UV radiation to continue to act on the surface of the tool 702. Furthermore, similar to the reflective cap 604, the reflected UV radiation may travel in a different path through the small compartment 306 impinging on different portions of the tool 702.

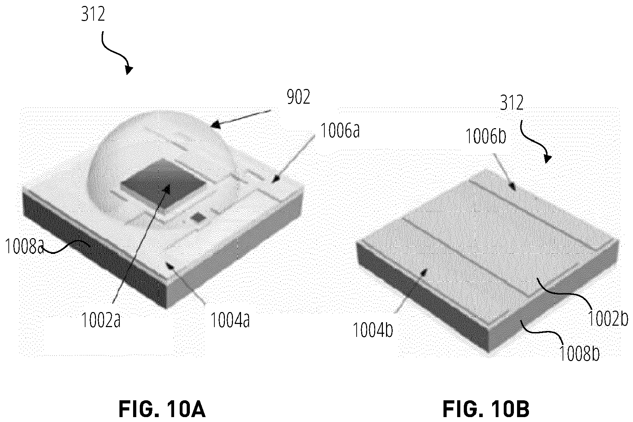

FIGS. 10A and 10B illustrate embodiments of a UV light 312 from the bottom light panel 310. The UV lights 316 of the top light panel 314 may be substantially the same as the UV lights 312 from the bottom light panel 310.

FIG. 10A illustrates an LED 1008a including a cover material 902. As described above, the cover material 902 may be formed from a UV transparent material and may be configured to act as an optical device to spatially distribute or concentrate the light leaving the LED 1008a. The LED 1008a may include an anode 1004a and a cathode 1006a. The LED 1008a may be operatively connected to a power source through the anode 1004a and the cathode 1006a. For example, a positive connection from the power source may be operatively connected to the anode 1004a and a negative connection from the power source may be operatively connected to the cathode 1006a. The power from the power source passing through the LED 1008a from the anode 1004a to the cathode 1006a may cause the UV light source 1002a to emit UV radiation. The LED 1008a and UV light source 1002a of the LED 1008a may be configured to emit UV radiation in the UVB and UVC spectrums.

FIG. 10B illustrates an LED 1008b without a cover material 902. The LED 1008b may include an anode 1004b and a cathode 1006b. The LED 1008b may be operatively connected to a power source through the anode 1004b and the cathode 1006b. For example, a positive connection from the power source may be operatively connected to the anode 1004b and a negative connection from the power source may be operatively connected to the cathode 1006b. The power from the power source passing through the LED 1008b from the anode 1004b to the cathode 1006b may cause the UV light source 1002b to emit UV radiation. The LED 1008b and UV light source 1002b of the LED 1008b may be configured to emit UV radiation in the UVB and UVC spectrums.

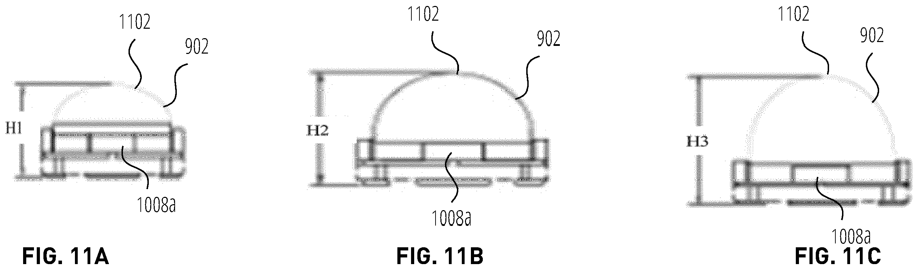

FIGS. 11A, 11B, and 11C illustrate different configurations of an LED 1008a including a cover material 902 similar to FIG. 10A. As shown in FIG. 9 multiple reflections on the walls 802 of the compartments 304, 306 may be desirable. The cover material 902 may be configured as an optical lens 1102, which may be designed to emit UV radiation at a targeted beam angle or view angle that may be chosen to fit a form factor of the tool 702 or surfaces to be treated.

A lens height of the lens 1102 may control the target beam angle of the UV radiation after passing through the lens 1102. For example, the LED 1008a illustrated in FIG. 11A may have a lens height H1 between about 2.00 mm and about 2.10 mm, such as about 2.06 mm. The resulting view angle may be between about 110 degrees and about 130 degrees. In the embodiment of the LED 1008a illustrated in FIG. 11B, the lens height H2 may be between about 2.50 mm and about 2.60 mm, such as about 2.56 mm and the view angle may be between about 70 degrees and about 90 degrees. In the embodiment of FIG. 11C, the lens height H3 may be between about 3.05 mm and about 3.15 mm such as about 3.11 mm and the view angle may be between about 40 degrees and about 60 degrees.

The target beam angle of the respective LED 1008a may be selected based on the types of tools 702 being sanitized and/or a size or shape of the associated compartments 304, 306. For example, the target beam angle of the respective LED 1008a may affect the distribution of the UV radiation within the respective compartments 304, 306. The target beam angle may also affect the effectiveness of the UV radiation throughout the respective compartments 304, 306. In some embodiments, the UV lights 312, 316 associated with the different compartments 304, 306 of the same sterilization apparatus 200 may include lenses 1102 having different heights configured to adjust the target beam angle of the respective UV lights 312, 316 to the associated compartments 304, 306.

Embodiments of the present disclosure may enable sterilization processes to take place quickly and in a small area. Sterilizing tools in smaller areas and more quickly may enable a medical professional to position a sterilization apparatus in an area near where the medical tools are used, such that the tools may be sterilized quickly between patients rather than requiring multiple different sets of tools for each examination area. This may reduce the number of tools that a medical office may need to maintain, which may reduce costs associated with maintaining a medical practice.

Embodiments of the present disclosure may sterilize tools causing the materials of the tools to break down or deteriorate. Thus, embodiments of the present disclosure may extend the life of the associated medical tools. The medical tools that are typically going through sanitization processes may be expensive tools. Therefore, extending the life of the medical tools may reduce the expenses of the associated medical professionals or medical offices.

Non-limiting example embodiments of the present disclosure may include:
Embodiment 1: A sterilization apparatus comprising: a cavity defined by walls of the apparatus; at least one internal compartment within the cavity, the internal compartment comprising at least one reflective surface; one or more ultraviolet lights on a surface of at least one of the walls of the apparatus and directed toward the at least one internal compartment, the one or more ultraviolet lights configured to produce ultraviolet light in one or more of a UVB spectrum and a UVC spectrum.
Embodiment 2: The sterilization apparatus of embodiment 1, wherein the at least one internal compartment comprises a small internal compartment.
Embodiment 3: The sterilization apparatus of embodiment 2, wherein at least one of the one or more ultraviolet lights is positioned at an axial end of the small internal compartment and directed into the small internal compartment.
Embodiment 4: The sterilization apparatus of any one of embodiments 2 or 3, wherein the at least one reflective surface comprises an internal surface of the small internal compartment.
Embodiment 5: The sterilization apparatus of any one of embodiments 2 through 4, wherein the small internal compartment comprises a cap configured to be positioned on an axial end of the small internal compartment.
Embodiment 6: The sterilization apparatus of embodiment 5, wherein the cap comprises a coupler configured to couple a tool to the cap and hold and suspend the tool within the small internal compartment from the cap.
Embodiment 7: The sterilization apparatus of any one of embodiments 5 or 6, wherein the coupler comprises a UV transparent material.
Embodiment 8: The sterilization apparatus of any one of embodiments 5 through 7, wherein the coupler comprises a UV reflective material.
Embodiment 9: The sterilization apparatus of any one of embodiments 2 through 8, wherein the small internal compartment comprises at least one of a cylindrical tube, conical tube, pyramidal tube, or polyhedron tube with a vertical axis and axial line of sight for at least one of the one or more ultraviolet lights.
Embodiment 10: The sterilization apparatus of any one of embodiments 1 through 9, wherein the at least one cavity comprises a support configured to suspend a device within the at least one cavity and allow light from the one or more ultraviolet lights to pass through the support and impinge on a surface of the device through the support.
Embodiment 11: The sterilization apparatus of any one of embodiments 1 through 10, wherein the at least one reflective surface comprises at least one of polytetrafluoroethylene, stainless steel, aluminum, nickel, titanium, chromium or gold.
Embodiment 12: A sterilization appliance comprising: a bottom surface; a lid; one or more walls; a cavity defined by the bottom surface, the lid, and the one or more walls; at least one large compartment positioned within the cavity; one or more small compartments positioned within the cavity; and a plurality of UV light sources positioned on at least one of the bottom surface, the lid, or the one or more walls, the plurality of UV lights positioned to correspond with at least one of the at least one large compartment and the one or more small compartments.
Embodiment 13: The sterilization appliance of embodiment 12, wherein at least one of the plurality of UV light sources is positioned to correspond with each of the at least one of the at least one large compartment and the one or more small compartments.
Embodiment 14: The sterilization appliance of any one of embodiments 12 or 13, wherein at least two of the plurality of UV light sources are positioned to correspond to the at least one large compartment.
Embodiment 15: The sterilization appliance of any one of embodiments 12 through 14, wherein the bottom surface comprises a removable drawer.
Embodiment 16: The sterilization appliance of any one of embodiments 12 through 15, wherein the plurality of UV light sources are positioned on the lid.
Embodiment 17: The sterilization appliance of any one of embodiments 12 through 16, wherein the plurality of UV light sources comprise a lens configured to emit UV radiation from the UV light sources at a targeted beam angle.
Embodiment 18: A method of sterilizing a tool comprising: suspending the tool in an internal compartment, the compartment comprising a UV reflective surface; closing a lid over the internal compartment; and supplying UV radiation to the internal compartment at a beam angle configured to reflect multiple times from the UV reflective surface of the compartment.
Embodiment 19: The method of embodiment 18 wherein the UV radiation comprises UV radiation in at least one of a UVB spectrum or a UVC spectrum.
Embodiment 20: The method of any one of embodiments 18 or 19, wherein suspending the tool comprises coupling the tool to a support within the internal compartment.

The embodiments of the disclosure described above and illustrated in the accompanying drawing figures do not limit the scope of the invention, since these embodiments are merely examples of embodiments of the invention, which is defined by the appended claims and their legal equivalents. Any equivalent embodiments are intended to be within the scope of this disclosure. Indeed, various modifications of the present disclosure, in addition to those shown and described herein, such as alternative useful combinations of the elements described, may become apparent to those skilled in the art from the description. Such modifications and embodiments are also intended to fall within the scope of the appended claims and their legal equivalents.

## Claims

1. A sterilization apparatus comprising:
a cavity defined by walls of the apparatus;
at least one internal compartment within the cavity, the internal compartment comprising at least one reflective surface;
one or more ultraviolet lights on a surface of at least one of the walls of the apparatus and directed toward the at least one internal compartment, the one or more ultraviolet lights configured to produce ultraviolet light in one or more of a UVB spectrum and a UVC spectrum.

2. The sterilization apparatus of claim 1, wherein the at least one internal compartment comprises a small internal compartment.

3. The sterilization apparatus of claim 2, wherein at least one of the one or more ultraviolet lights is positioned at an axial end of the small internal compartment and directed into the small internal compartment.

4. The sterilization apparatus of claim 2, wherein the at least one reflective surface comprises an internal surface of the small internal compartment.

5. The sterilization apparatus of any one of claims 2 through 4, wherein the small internal compartment comprises a cap configured to be positioned on an axial end of the small internal compartment.

6. The sterilization apparatus of claim 5, wherein the cap comprises a coupler configured to couple a tool to the cap and hold and suspend the tool within the small internal compartment from the cap.

7. The sterilization apparatus of claim 5, wherein the coupler comprises a UV transparent material.

8. The sterilization apparatus of claim 5, wherein the coupler comprises a UV reflective material.

9. The sterilization apparatus of any one of claims 2 through 4, wherein the small internal compartment comprises at least one of a cylindrical tube, conical tube, pyramidal tube, or polyhedron tube with a vertical axis and axial line of sight for at least one of the one or more ultraviolet lights.

10. The sterilization apparatus of any one of claims 1 through 4, wherein the at least one cavity comprises a support configured to suspend a device within the at least one cavity and allow light from the one or more ultraviolet lights to pass through the support and impinge on a surface of the device through the support.

11. The sterilization apparatus of any one of claims 1 through 4, wherein the at least one reflective surface comprises at least one of polytetrafluoroethylene, stainless steel, aluminum, nickel, titanium, chromium or gold.

12. The sterilization appliance of any one of claims 1 through 4, wherein the one or more ultraviolet lights comprise a lens configured to emit UV radiation from the one or more ultraviolet lights at a targeted beam angle.

13. A method of sterilizing a tool comprising:
suspending the tool in an internal compartment, the compartment comprising a UV reflective surface;
closing a lid over the internal compartment; and
supplying UV radiation to the internal compartment at a beam angle configured to reflect multiple times from the UV reflective surface of the compartment.

14. The method of claim 13 wherein the UV radiation comprises UV radiation in at least one of a UVB spectrum or a UVC spectrum.

15. The method of claim 13, wherein suspending the tool comprises coupling the tool to a support within the internal compartment.
